# FASCICULE DE BREVET EUROPEEN

(11) **EP 0 918 540 B1**
(45) Date de publication et mention de la délivrance du brevet: **25.09.2002**
(21) Numéro de dépôt: 97934575.8
(22) Date de dépôt: 16.07.1997
(51) Int. Cl.: A61K 39/155, A61K 39/265, A61D 7/00

(54) **VACCIN POLYNUCLEOTIDIQUE BOVIN POUR VOIE INTRADERMIQUE**
INTRADERMALER POLYNUKLEOTID-IMPFSTOFF AUS RIND
INTRADERMAL BOVINE POLYNUCLEOTIDE VACCINE

(30) Priorité: 19.07.1996 FR 9609402
(43) Date de publication de la demande: 02.06.1999
(73) Titulaire: MERIAL, 69002 Lyon (FR); ID-DLO Institute of Animal Science and Health, 8200 AB Lelystad (NL)
(72) Inventeur: RIJSEWIJK, Franciscus, Antonius, Maria, NL-1017 JP Amsterdam (NL); SCHRIJVER, Remco, Siebren, NL-3722 JH Bilthoven (NL); VAN OIRSCHOT, Johannes, Theodorus, NL-8212 AN Lelystad (NL)
(74) Mandataire: Colombet, Alain André
(86) Numéro de dépôt international: FR9701322
(87) Numéro de publication internationale: WO98003196

(56) Documents cités:
- WO-A-92/01471
- WO-A-95/20660
- FR-A- 2 348 709
- RAZ E. ET AL.: "Intradermal gene immunization: The possible role of DNA uptake in the induction of cellular immunity to viruses" PROCEEDINGS OF THE NATIONAL ACADEMY OF SCIENCES OF USA, vol. 91, no. 20, 27 septembre 1994, WASHINGTON US, pages 9519-9523, XP002028211 cité dans la demande
- FURTH P.A. ET AL.: "Gene Transfer into Somatic Tissues by Jet Injection" ANALYTICAL BIOCHEMISTRY, vol. 205, 1992, NEW YORK US, pages 365-368, XP000647725 cité dans la demande
- VAHLSING H.L. ET AL.: "Immunization with plasmid DNA using a pneumatic gun" JOURNAL OF IMMUNOLOGICAL METHODS, vol. 175, 1994, NEW YORK US, pages 11-22, XP002028212 cité dans la demande

## Description

La présente invention concerne un perfectionnement apporté à la vaccination des bovins.

L'immunisation et la vaccination par administration directe de séquences nucléotidiques codant pour une protéine immunogène (dite vaccination polynucleotidique ou par ADN) a été décrite dans la demande de brevet WO-A-90 11092. La protéine codée par la séquence nucléotidique insérée est susceptible d'être exprimée dans les cellules et d'entraîner le développement d'une réponse immunitaire. Cette demande prévoit l'utilisation d'ADN nu ainsi que d'ADN contenu dans des liposomes. De façon préférée, l'ADN est introduit dans le muscle. L'ADN pourrait aussi être introduit dans la peau, dans certains organes ou dans le sang, l'injection pouvant être réalisée de différentes manières telles que la voie intradermique, la voie transcutanée, la voie intraveineuse, etc.

Les travaux qui ont suivi les premières descriptions de cette technique ont démontré l'intérêt d'utiliser soit la voie intramusculaire pour l'injection de l'ADN, soit la méthode dite "gene gun" qui consiste à propulser des microparticules métalliques, telles que des microparticules d'or, revêtues de l'ADN directement dans les couches cellulaires superficielles de la peau.

J.B. ULMER et al., Science, Volume 259, 19 mars 1993, 1745-1749 ; G.J.M. COX et al., J. of Virology, Volume 67, n° 9, septembre 1993, 5664-5667 ; Z.Q. XIANG dans Virology 199, 132-140 (1994), ont décrit des essais de vaccination par ADN en utilisant la voie intramusculaire.

Il a également été largement démontré que la voie intramusculaire donnait des résultats supérieurs à la voie intradermique, mais que, en fin de compte, la voie la plus prometteuse était l'utilisation du "gene gun", car, avec cette technique, les doses administrées sont très inférieures aux doses nécessaires par voie intramusculaire. On peut se référer à F. FYNAN et al. dans P.N.A.S. USA Volume 90, 11478-11482, Décembre 1993, WO-A-95/20660.

De même D. TANG et al. (Nature 356, 152-154, 12 mars 1992) ont montré l'absence de réponse immunitaire après l'administration d'hormone de croissance humaine par voie intradermique à l'aide d'une aiguille hypodermique. Les auteurs ont en revanche démontré l'obtention d'une réponse immunitaire à l'aide de la technique du "gene gun".

Seuls E. RAZ et al. (P.N.A.S. USA, Vol. 91, 9519-9523, septembre 1994) ont rapporté que l'administration intradermique pouvait induire des titres en anticorps élevés.

De son côté, la technique du "gene gun" a l'inconvénient d'être difficile et coûteuse à mettre en oeuvre, puisqu'elle nécessite la préparation et l'utilisation de particules d'or revêtues de l'ADN et leur administration à l'aide d'un propulseur spécial.

Des auteurs ont donc développé une technique alternative qui prévoit l'utilisation d'un appareil d'administration par jet liquide. On peut citer P.A. FURTH et al. dans Analytical Biochemistry 205, 365-368, 1992 qui décrivent l'utilisation de l'appareil dénommé Ped-o-jet, qui est un injecteur utilisé pour délivrer des vaccins humains dans les tissus musculaires, pour l'administration d'un vaccin ADN. Les auteurs rapportent que l'injecteur peut faire passer l'ADN à travers la peau et atteindre les muscles, le tissu graisseux et le tissu mammaire des animaux vivants.

H.L. VAHLSING et al., dans Journal of Immunological Methods 75 (1994) 11-22, décrivent l'utilisation de l'appareil dénommé Med-E-Jet pour une administration transcutanée et intramusculaire.

On peut citer encore M. JENKINS et al., dans Vaccine 1995, Volume 13, n° 17, 1658-1664, qui décrivent l'utilisation de la vaccination par jet dans le muscle.

Le virus syncitial respiratoire bovin BRSV est présent dans le monde entier et peut causer des maladies sévères du tractus respiratoire inférieur chez les bovins, cette maladie étant similaire à celle causée par le virus respiratoire syncytial HRSV chez les enfants. Au cours d'une étude, il a été trouvé que plus de 95 % des veaux âgés de 2 ans étaient infectés par le virus BRSV (Van der Poel et al., Archives of Virology 1993, 133, 309-321).

Le besoin en vaccin contre le virus BRSV est ressenti mais n'a pas donné lieu au développement de vaccins efficaces. Les premières tentatives de vaccination des enfants a conduit à l'apparition d'une facilitation de la maladie après infection naturelle, suggérant que la vaccination pouvait être dangereuse (Anderson et al., Journal of Infectious Diseases, 1995, 171 : 1 à 7). Il est connu cependant que des anticorps contre les deux glycoprotéines majeures de surface, (F protéine de fusion) et G (protéine d'attachement) pourraient jouer un rôle dans la protection (Kimman et Westenbrink, Archives of Virology, 1990, 112 : 1 à 25). De nombreux travaux ont également été menés sur des modèles d'animaux de type souris, chien, furet. En revanche, les essais de vaccination sur bovin avec la protéine F purifiée n'ont pas donné de résultat concluant, dans la mesure où comme chez les enfants vaccinés par HRSV, les veaux ont développé des anticorps neutralisants et des anticorps non neutralisants pouvant interférer avec la réponse immunitaire lors d'une infection ultérieure par le virus (L.D. Nelson et al., Am. J. Vet. Res., Vol. 53, n° 8, août 1992, p 1315-1321).

La présente invention a pour objectif de fournir un perfectionnement à la vaccination des bovins par ADN qui permette d'assurer une vaccination au moins aussi efficace que la vaccination par voie intramusculaire ou par la technique du "gène gun" mais qui soit de mise en oeuvre plus aisée et moins coûteuse.

Un autre objectif de l'invention est de fournir un tel perfectionnement conduisant à une innocuité accrue, essentiellement en ce qui concerne les résidus de vaccination présents dans les tissus.

Un autre objectif de l'invention, qui regarde la vaccination des animaux destinés à la consommation, est d'assurer également une innocuité telle que la vaccination ne présente pas d'effets défavorables sur la présentation de la viande.

Un autre objectif de l'invention est de fournir un moyen de vaccination de masse.

Un objectif spécifique de l'invention est de fournir un tel vaccin permettant la protection des bovins contre le virus BRSV, le virus IBR, le virus BVD ou le virus PI-3.

Les demandeurs ont trouvé qu'il était possible de remplir ces objectifs en administrant le vaccin par voie intradermique à l'aide d'un injecteur liquide sans aiguille, assurant en 5 points une répartition du vaccin essentiellement dans l'épiderme, le derme et l'hypoderme Les essais conduits par les demandeurs dans le domaine de la vaccination des bovins contre le virus respiratoire syncitial bovin (Bovine Respiratory Syncitial Virus, BRSV) ont permis d'obtenir des résultats d'immunisation supérieurs par cette voie à ce qui est obtenu par la voie intramusculaire.

La présente invention propose pour la première fois la vaccination des bovins par des vaccins polynucléotidiques (ou vaccins ADN ou encore vaccins plasmidiques) conçus pour, et administrés par la voie intradermique au moyen d'un injecteur sans aiguille par jet liquide.

La présente invention a donc pour objet une formule de vaccin polynucléotidique comprenant une quantité efficace par voie intradermique d'un plasmide associant une séquence d'ADN codant pour un immunogène et un promoteur permettant l'expression de cet immunogène in vivo dans les cellules de la peau, cette formule de vaccin étant adaptée à l'administration intradermique (on vise en particulier les cellules de l'épiderme, du derme et de l'hypoderme ; l'administration vise en particulier à présenter les antigènes exprimés aux cellules dendritiques de Langerhans de la peau, lesquelles se localisent essentiellement dans l'épiderme) par un appareil d'administration intradermique par jet liquide, en particulier l'appareil dénommé Pigjet (fabriqué et distribué par Endoscoptic, Laons, France) ou un appareil équivalent délivrant le vaccin par une tête à 5 buses dans des conditions équivalentes au Pigjet. De manière générale, les formules de vaccin selon l'invention seront adaptées pour une administration par un appareil d'administratrion par jet liquide possédant de 1 à 10 buses, de préférence de 4 à 6, plus préférentiellement encore de 5 à 6.

Cela passe par un véhicule adapté à la voie intradermique, tel que eau, tampon, eau physiologique, liposomes, lipides cationiques et en général véhicule de faible viscosité, notamment équivalence ou proche de celle de l'eau, et par un volume de dose utile et efficace par cette voie.

Notamment, mais pas exclusivement, avec un appareil ayant 5 ou 6 buses, c'est-à-dire administrant la dose par 5 ou 6 orifices et sous forme de 5 ou 6 jets de volumes identiques, le volume de dose pourra être avantageusement compris entre 0,1 ml et 0,9 ml, de préférence entre 0,2 et 0,6 ml, préférentiellement de l'ordre de 0,4 à 0,5 ml.

La formule de vaccin comprendra une quantité de plasmide intradermiquement efficace qui sera en général de 10 ng à 1 mg, de préférence de 100 ng à 500 µg, préférentiellement de 0,5 µg à 50 µg de plasmide.

Typiquement, l'invention cherche à administrer la formule de vaccin en plusieurs points afin d'optimiser la transfection des cellules par les plasmides. Cela se traduit par une préférence pour l'usage d'une tête d'éjection à plusieurs trous. Cela peut aussi être combiné à une multi-application de l'appareil, c'est-à-dire à la répartition de la dose vaccinale en plus d'une application de l'appareil en des endroits différents. De manière particulièrement préférée, on pourra utiliser un appareil à 5 ou 6 trous en mono-application ou en multi-application, de préférence en double-application.

Un cas typique de l'invention est une séquence d'ADN codant pour un immunogène du virus BRSV et en particulier pour le gène G et/ou F de ce virus (par exemple Souche 391-2 : R. Lerch et al. Virology. 1991. 181. 118-131).

Un autre cas typique de l'invention est une séquence d'ADN codant pour un immunogène du virus de la rhinotrachéite infectieuse bovine (IBR) ou herpèsvirus bovin (BHV), en particulier pour le gène gB et/ou le gène gD (par exemple Souche ST : Leung-Tack P. et al. Virology. 1994. 199. 409-421).

Un autre cas typique de l'invention est une séquence d'ADN codant pour un immunogène du virus de la maladie des muqueuses (BVD), en particulier le gène E2 et/ou le gène E1 (par exemple Souche Osloss : L. De Moerlooze et al. J. Gen. Virol. 1993. 74. 1433-1438). On peut également associer des gènes de différents sous-types de BVD, par exemple d'Amérique du Nord et d'Europe (A. Dekker et al., Veternary Microbiol., 1995, 47, 317-329).

Un autre cas typique encore de l'invention est une séquence d'ADN codant pour un immunogène du virus parainfluenza de type 3 (PI-3), en particulier pour le gène HN et/ou le gène F, de préférence le gène HN (séquence des gènes F et HN déposée par H. Shibuta en 1987. N° d'accès de la séquence sur GenBank = Y00115).

En cas de combinaison de 2 gènes, par exemple F et G de BRSV, HN et F de PI-3 ou gD et gB d'IBR, les séquences correspondantes pourront être insérées dans le même plasmide ou dans des plasmides différents.

Par gène d'agent pathogène, on entend non seulement le gène complet, mais aussi les séquences nucléotidiques différentes, y compris fragments, conservant la capacité à induire une réponse protectrice. La notion de gène recouvre les séquences nucléotidiques équivalentes à celles décrites précisémment dans les exemples, c'est-à-dire les séquences différentes mais codant pour la même protéine. Elle recouvre aussi les séquences nucléotidiques d'autres souches du pathogène considéré, assurant une protection croisée ou une protection spécifique de souche ou de groupe de souche. Elle recouvre encore les séquences nucléotidiques qui ont été modifiées pour faciliter l'expression in vivo par l'animal hôte mais codant pour la même protéine.

On comprend bien entendu que l'invention consiste en l'adaptation des vaccins ADN de l'art antérieur à une administration intradermique par un appareil d'administration par jet liquide. Si cela entraîne des modifications au niveau de la formule de vaccin et en particulier de la viscosité, de la quantité d'ADN et du volume de dose à administrer, il va de soi que l'invention s'applique par ailleurs à toutes les constructions de vaccin ADN décrites dans l'art antérieur. L'homme de l'art pourra donc se référer à l'état de l'art du domaine de la vaccination ADN et en particulier aux documents discutés plus haut.

De manière plus spécifique, les unités de transcription utilisées dans les formules de vaccin selon l'invention comprendront un promoteur eucaryote fort, tel que le promoteur hCMV IE.

La formule de vaccin selon l'invention peut être conditionnée dans un flacon multi-doses, par exemple de 10 à 100 doses, adapté à un appareil d'administration intradermique par jet liquide, de préférence le Pigjet.

La présente invention a encore pour objet une installation portative de vaccination bovine comprenant un appareil d'administration par jet liquide et un flacon adapté comprenant plusieurs doses d'une formule de vaccin tel que décrit précédemment, l'appareil d'administration étant conçu pour délivrer une dose de formule de vaccin en intradermique.

De préférence, l'appareil d'administration comporte une tête d'éjection munie de 1 à 10 buses, notamment de 4 à 6, de préférence 5 ou 6.

L'appareil d'administration peut avoir les différentes caractéristiques données dans la description détaillée. Les appareils d'administration préférés sont ceux qui reproduisent les conditions d'administration obtenues avec l'appareil Pigjet.

La présente invention a également pour objet l'utilisation d'un plasmide associant une séquence d'ADN codant pour un immunogène de pathogène bovin et un promoteur permettant l'expression de ce type d'immunogène, pour la préparation d'une formule de vaccin polynucléotidique selon les différents modalités décrites ci-dessus, adaptée à l'administration intradermique par un appareil d'administration par jet liquide.

L'invention a également pour objet une méthode de vaccination dans laquelle on administre par voie intradermique à l'aide d'un appareil d'administration par jet liquide, une formule de vaccin polynucléotidique telle que décrite ci-dessus. L'administration du vaccin peut s'effectuer par une ou plusieurs décharges d'un volume de formule déterminé. De même, il est possible de prévoir une ou plusieurs vaccinations réparties dans le temps.

La méthode de vaccination selon l'invention pourra reprendre les données citées plus haut en ce qui concerne notamment l'appareil d'administration à utiliser.

L'invention va être maintenant décrite plus en détails à l'aide de modes de réalisation de l'invention non limitatifs en se référant aux dessins annexés dans lesquels :
- la figure 1 décrit un plasmide comprenant le gène G du virus BRSV sous le contrôle du promoteur hCMV, pour la vaccination des bovins contre ce virus.
- la figure 2 représente un graphe comparant l'efficacité d'une vaccination contre le virus BRSV par voie intramusculaire IM (bovin 1349) et intradermique ID (bovins 1352 et 1353), avec les jours en abcisse et le titre infectieux en ordonnée.

### Préparation du gène synthétique G de BRSV

Le virus syncitial respiratoire bovin est un Pneumovirus de la famille des Paramyxovirus. Il s'agit d'un virus enveloppé qui se réplique dans le cytoplasme et possède un ARN génomique simple brin en orientation négative. Le génome de BRSV code pour deux glycoprotéines transmembranaires majeures, la protéine de fusion F et la protéine d'attachement G.

On a réalisé une version synthétique du gène G en retirant de ce gène tous les signaux d'épissage potentiels (G. Keil, Federal Research Centre for Virus Diseases of Animals, Friedrich Löffler Institute, D-17498, Insel Riems, Germany).

La région codant pour le gène G a été déterminée par Lerch et al., Journal of Virology, 1990, 64, 5559-5569). La région codante correspond à 257 acides aminés et à la caractéristique d'une glycoprotéine transmembranaire de type II. Elle possède un domaine cytoplasmique N-terminal de 40 acides aminés suivi d'une région transmembranaire de 25 acides aminés. Le restant, à savoir les 192 acides aminés C-terminaux, forment le domaine extra-cellulaire de la protéine G. On a synthétisé une séquence d'ADN ayant un cadre ouvert de lecture codant pour exactement les mêmes 257 acides aminés trouvés par Lerch et al, mais sans les signaux d'épissage potentiels. On a réalisé la traduction inverse de la séquence des 257 acides aminés en toutes les séquences d'ADN possibles codant pour une telle séquence d'acides aminés. Cela a été réalisé en utilisant le programme de traduction inverse d'une séquence de protéine RTRANS de PCGene de A. Bairoch., Université de Genève, Suisse (IntelliGenetics Inc). On a déterminé les sites potentiels d'épissage en utilisant le programme d'analyse d'acide nucléique "Signal". Ce programme est basé sur la méthode de la matrice pondérée de Staden (1984, Nucleic Acids Research 12, 505-519). Ce programme identifie les sites donneurs potentiels d'épissage (bordures intron/exon) et les sites accepteurs potentiels d'épissage (bordures exon/intron). A l'aide de ces données de séquence, on a pu muter l'ensemble des signaux d'épissage forts potentiels sans modifier la capacité pour la protéine. En particulier, les dinucléotides GT qui peuvent former l'extrémité 5' d'un intron et les dinucléotides AG qui peuvent former l'extrémité 3' d'un intron ont été retirés lorsque cela était possible.

Pour synthétiser la séquence de nucléotidiques adaptée codant pour G, on a synthétisé des oligonucléotides d'environ 100 résidus couvrant les deux brins de la séquence complète, à l'aide d'un synthétiseur d'ADN (par exemple synthétiseur d'ADN, Perkin, Elmers/Applied Biosystems 381A ; on peut également utiliser les oligonucléotides commercialement disponibles). Les oligonucléotides complémentaires sont hybridés pour former un fragment double brin et clonés dans des vecteurs procaryotes tels que pUC18 ou pUC19. En utilisant des sites de restriction enzymatique appropriés, on lie ensemble les fragments d'ADN clonés dans le bon ordre en utilisant les procédures de clonage standard (Sambrook et al., Molecular Cloning : A laboratory Manual, 2ème édition, Cold Spring Harbor Laboratory, Cold Spring Harbor New York 1989). La séquence nucléotidique du fragment d'ADN qui en résulte a été déterminée à l'aide des procédures de séquençage standard (Sambrook et al.) pour contrôler que la séquence est correcte.

### Construction du vecteur d'expression eucaryote comprenant le gène G derrière le promoteur hCMV.

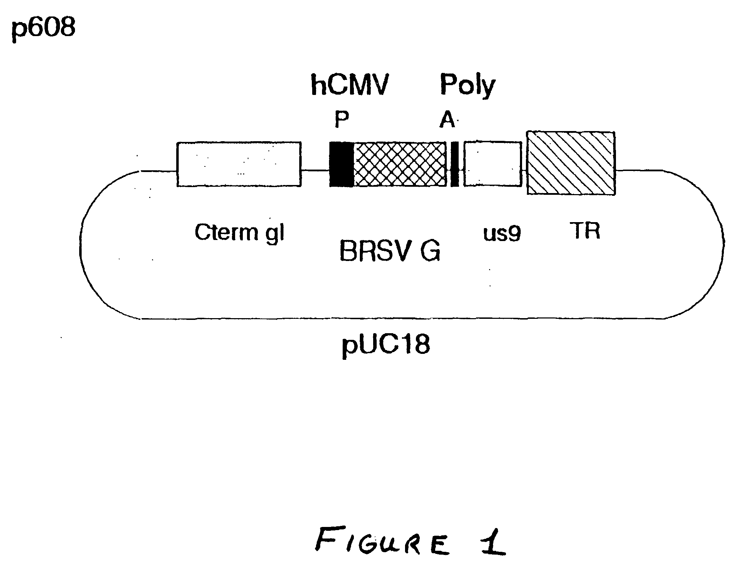

Pour assurer son expression, le gène synthétique G a été cloné dans un vecteur. Dans cet essai préliminaire, on a utilisé un vecteur immédiatement disponible au laboratoire, à savoir le vecteur d'expression eucaryote 175hCMV. Ce vecteur plasmidique contient deux fragments provenant du virus BHV1 qui flanquaient à l'origine la glycoprotéine E de la souche BHV1 hollandaise appelée Lam (Van Engelenburg et al., 1994, Journal of General Virology 75, 2311-2318). Le fragment flanquant gauche (Cterm gI) commence au site PstI situé dans le cadre de lecture ouvert de la glycoprotéine gI et se termine au site BstBI (ou AsuII), 17 nucléotides en amont du début (start) du cadre ouvert de lecture du gène gE. Ce fragment est indiqué par : Cterm gI. Le fragment flanquant droit commence au site EcoNI situé au niveau du codon stop du cadre ouvert de lecture gE et se termine au niveau du premier site SmaI amont dans le fragment de répétition terminal (TR). Ce fragment code pour le gène US9. Ce fragment est indiqué par : US9 et TR. Ces deux fragments ont été clonés dans le site PstI et le site EcoRI (bout franc) de pUC18. Entre le site BstBI et le site EcoNI, un fragment AseI (bout franc) de 720 pb contenant la plus grande partie du promoteur Immediate Early du cytomégalovirus humain (hCMV-P) a été cloné avec une région polylinker et se terminant avec un signal de polyadénylation (Poly A). Le gène G synthétique a été cloné dans l'orientation indiquée à l'intérieur du site SmaI de cette région polylinker. Le plasmide obtenu a été appelé PR608. Voir la figure 1.

### Contrôle de l'expression in vitro de G à partir du plasmide PR608 :

On a réalisé un essai d'expression transitoire afin de tester si le plasmide PR608 peut exprimer la protéine G. Pour cet essai, 1,5 µg d'ADN purifié de plasmide PR608 a été transfecté dans une monocouche de cellules de trachée embryonnaire bovine (EBTr) en culture. Ces cellules EBTr ont été cultivées dans du milieu essentiel minimal de Earle avec 10 % de sérum bovin foetal (Integro) et des antibiotics [125 UI de pénicilline (Gist-Brocades), 125 µg de streptomycine (Biochemie), 37,5 UI de nystatin (Sigma) et 37,5 µg de kanamycine (Sigma) par ml]. La transfection a été réalisée conformément à la méthode standard de précipitation au phosphate de calcium selon F.L. Graham et A.J. van der Eb (1973, Virology 52, 456-467). Après transfection, l'ADN plasmidique est transporté dans le noyau des cellules transfectées et les protéines codées sont exprimées grâce au mécanisme de la cellule hôte. Normalement, seulement de 0,01 % à 0,1 % des cellules de la monocouche transfectée vont internaliser l'ADN et exprimer les gènes codés.

### Préparation de l'ADN pour la vaccination polynucléotidique :

L'ADN du plasmide PR608 a été préparé en ajoutant 100 µl d'un stock d'E. coli K12 dans le glycérol, cellules DH5alpha F-comprenant le plasmide PR608 à 2 litres de milieu LB, qui est un milieu standard pour la croissance des bactéries. Cette culture de 2 litres est incubée à 37°C pendant 20 heures et les cellules amplifiées sont récoltées en utilisant des flacons de 250 ml et une centrifugeuse IEC Centra-8R à vitesse maximale. L'ADN du plasmide PR608 a été isolé des cellules du culot en suivant la méthode de lyse alcaline standard de Birnboim et Doly telle que décrite par Sambrook et al., (1989, Molecular Cloning, a Press Laboratory Manual, 2ème Ed., Cold Spring Harbor Laboratory Press). L'ADN du plasmide isolé a été ensuite dissous dans 20 ml de TE (10 mM Tris et 1 mM EDTA, pH 7,4) et soumis à une électrophorèse pour déterminer la concentration d'ADN et pour estimer la qualité de l'ADN. A partir d'une culture normale de 2 litres, on peut isoler 40 mg de plasmides; environ 60 % du plasmide PR608 se trouve dans l'état super enroulé et environ 30 % et dans l'état circulaire relâché.

La préparation de plasmides est stockée à - 20°C. Avant application de l'ADN de plasmide PR608, la solution d'ADN est diluée à 0,5 mg/ml dans 1 x PBS et TE. Cette solution d'ADN tamponnée est pipetée dans des flacons de 10 ml adaptés à l'appareil Pigjet et stockés à 4°C pour une utilisation dans les 1 à 2 heures.

### Appareil d'administration Pigjet

L'appareil d'administration de type portatif, de type Pigjet, comprend dans un boitier muni d'une poignée, une chambre calibrée à 0,2 ml et un piston maintenu normalement en position rentrée dans la chambre par un ressort solidaire dudit piston.

L'appareil comporte en outre une tête à 5 gicleurs ou buses, destinés à calibrer le jet, soit une tête à 1 gicleur et un dispositif de filtration pour éviter l'injection d'éventuelles impuretés. Les gicleurs sont légèrement écartés les uns des autres.

La poussée du jet à la sortie du gicleur peut être fixée à 100 bars pour le Pigjet à 1 gicleur.

Provoquée par un moyen approprié, la compression du ressort provoque le déplacement du piston et donc l'aspiration de la dose de vaccin à partir d'un réservoir ou flacon adapté, fixé sur le boitier.

La libération du ressort provoque la rentrée du piston et l'éjection de la dose par la ou les buses.

### Essais de vaccination et d'épreuve :

4 veaux exempts d'organismes pathogènes spécifiques ont été obtenus par césarienne, ont été privés de collostrum et élevés à l'isolement. Les veaux ont été vaccinés 6 fois à 1 semaine d'intervalle, à partir de l'âge de 6 semaines. 2 animaux ont été vaccinés par voie intradermique au moyen du Pigjet à 1 gicleur (Endoscoptic, Laons, France) et 2 animaux ont été vaccinés par voie intramusculaire avec aiguille calibrée 25. Le Pigjet est appliqué de façon que sa tête soit au contact de la peau et perpendiculaire à celle-ci, afin que le jet de vaccin ait une direction orthogonale à la peau. Chaque vaccination a consisté en 500 µg d'ADN de plasmide dans 1 ml de tampon phosphate PBS. Les vaccinations intradermiques ont consisté en 5 injections de 0,2 ml dans la peau de la patte postérieure et les vaccinations intramusculaires ont consisté en 1 injection dans le muscle gluteus. La peau a été rasée avant vaccination.

On a obtenu les titres en anticorps qui apparaissent dans le tableau I suivant :
- vaccination aux semaines 0, 1, 2, 3, 4, 5
- épreuve à la semaine 6
- suivi du titre en anticorps aux semaines 0 à 8

| | 0 | 1 | 2 | 3 | 4 | 5 | 6 | 7 | 8 |
|---|---|---|---|---|---|---|---|---|---|
| veau 1352 Pigjet | <5 | <5 | <5 | 80 | 640 | 1280 | 640 | 1280 | >5120 |
| veau 1453 Pigjet | <5 | <5 | <5 | 40 | 1280 | 640 | 640 | 640 | ⁻^{a} |
| veau 1349 IM | <5 | <5 | <5 | <5 | <5 | <5 | <5 | <5 | <5 |
| veau 1350 IM | <5 | <5 | <5 | ^{a} | | | | | |

| | | | | | | | | | |
|---|---|---|---|---|---|---|---|---|---|
| a : ces veaux ont été sacrifiés en raison de l'apparition de problèmes de santé importants, non associés aux vaccinations. | | | | | | | | | |

Suite aux injections d'ADN, on observe l'apparition d'anticorps au bout de 3 semaines chez les animaux vaccinés par voie ID et de hauts titres à partir de la semaine 4 s'établissant en plateau.

Les veaux ont été ensuite éprouvés à l'aide de virus BRSV infectieux. L'inoculation du virus a été réalisée 6 semaines après la première vaccination par instillation intranasale d'1 ml de virus de souche Odijk (10^{3.8} TCID₅₀/ml) dans chaque naseau.

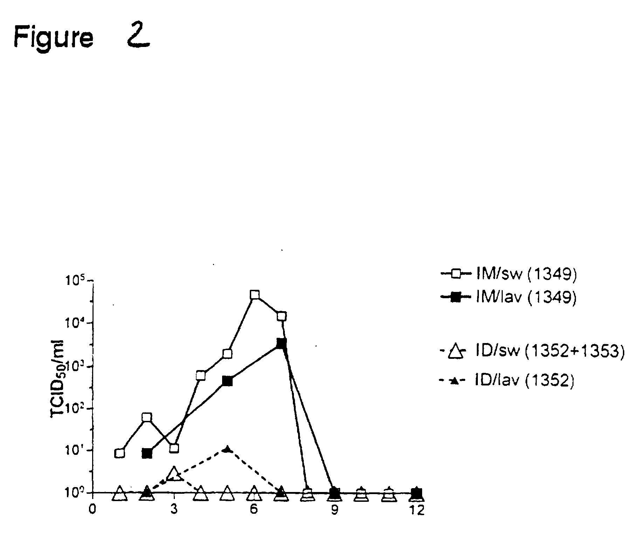

Pour estimer l'efficacité de la vaccination, on a suivi l'excrétion de virus par le titre infectieux TCID₅₀/ml jusqu'à 12 jours après l'infection, sur des écouvillons nasopharyngés (sw) et dans du fluide de lavage (Lav) des poumons (Figure 2).

On a pu démontrer qu'un plasmide codant pour la protéine G de BRSV administré par voie intradermique par jet liquide protégeait contre une épreuve virulente par ce virus. La vaccination intradermique à l'aide d'un appareil d'administration ayant une tête d'éjection à 1 trou, induit rapidement des titres en anticorps élevés contre la protéine G, ce qui n'a pas été observé par la vaccination intramusculaire. En outre, les titrages de virus BRSV infectieux dans les fluides de lavage des poumons et sur les écouvillons nasopharyngés étaient élevés pour les veaux vaccinés par voie intramusculaire et virtuellement absents chez les veaux vaccinés par voie intradermique. Les signes cliniques étaient légers et ne différaient pas de manière marquée entre les animaux.

On a également remarqué une très bonne innocuité lorsqu'on utilisait le Pigjet, cette technique ne nécessitant d'ailleurs pas d'induire une inflammation préalable du tissu comme c'est le cas avec les particules d'or.

En outre, ce qui est remarquable, c'est l'obtention d'une protection avec la protéine G qui, à l'ordinaire n'est pas très immunogène (P.L. Collins dans : The Paramyxoviruses, 1991, Ed. D.W. Kingsbury, New-York, Plénum Press) et le titre élevé en anticorps obtenu après vaccination intradermique est comparable à celui obtenu après infection naturelle ou vaccination avec un vaccin vivant testé par ailleurs.

L'invention concerne aussi la méthode de préparation des formules de vaccin telle qu'elle ressort de cette description.

## Revendications

1. Formule de vaccin polynucleotidique destiné au bovin comprenant une quantité efficace par voie intradermique d'un plasmide associant une séquence d'ADN codant pour un immunogène d'un agent pathogène bovin et un promoteur permettant l'expression de cet immunogène in vivo dans les cellules de la peau, cette formule de vaccin étant adaptée à l'administration intradermique par un appareil d'administration par jet liquide.

2. Formule de vaccin selon la revendication 1, **caractérisée en ce que** le plasmide est présenté dans un véhicule adapté à la voie intradermique sous un volume de dose compris entre 0,1 et 0,9 ml, de préférence entre 0,2 et 0,6 ml, plus préférentiellement entre 0,4 et 0,5 ml, apte à être administré par jet liquide en intradermique.

3. Formule de vaccin selon la revendication 1 ou 2, **caractérisée en ce que** le plasmide est présent dans la formule de vaccin en une quantité intradermiquement efficace de 10 ng à 1 mg, de préférence de 100 ng à 500 µg, plus préférentiellement de 0,5 µg à 50 µg.

4. Formule de vaccin selon la revendication 3, **caractérisée en ce que** la séquence d'ADN code pour un immunogène d'un agent pathogène choisi parmi le groupe consistant en : virus BRSV, virus IBR, virus BVD et virus PI 3.

5. Formule de vaccin selon la revendication 4, **caractérisée en ce que** la séquence d'ADN code pour le gène gB et/ou le gène gD du virus IBR.

6. Formule de vaccin selon la revendication 4, **caractérisée en ce que** la séquence d'ADN code pour le gène G et/ou F du virus BRSV.

7. Formule de vaccin selon la revendication 4, **caractérisée en ce que** la séquence d'ADN code pour E2 et/ou E1 du virus BVD.

8. Formule de vaccin selon la revendication 4, **caractérisée en ce que** la séquence d'ADN code pour HN et/ou F du virus PI 3.

9. Formule de vaccin selon l'une quelconque des revendications 1 à 8, **caractérisée en ce que** le promoteur est un promoteur eucaryote fort, tel que le promoteur hCMV IE.

10. Formule de vaccin selon l'une quelconque des revendications 1 à 9, **caractérisée en ce qu'**elle est conditionnée dans un flacon multi-doses adapté à un appareil d'administration intradermique par jet liquide, de préférence le Pigjet.

11. Installation portative de vaccination bovine comprenant un appareil d'administration par jet liquide et un flacon adapté comprenant plusieurs doses d'une formule de vaccin selon l'une quelconque des revendications 1 à 10, l'appareil d'administration étant conçu pour délivrer une dose de formule de vaccin en intradermique.

12. Installation selon la revendication 11, **caractérisée en ce que** l'appareil comporte une tête d'éjection munie de 1 à 10 buses, notamment de 4 à 6, de préférence 5 ou 6.

13. Utilisation d'un plasmide associant une séquence d'ADN codant pour un immunogène d'un agent pathogène bovin et un promoteur permettant l'expression de cet immunogène, pour la préparation d'une formule de vaccin polynucléotidique adaptée à l'administration intradermique par un appareil d'administration par jet liquide.

14. Utilisation selon la revendication 13, **caractérisée en ce qu'**elle comporte entre 10 ng et 1 mg d'ADN, de préférence entre 100 ng et 500 µg, de préférence entre 0,5 µg et 50 µg, sous un volume de dose compris entre 0,1 et 0,9 ml, de préférence entre 0,2 et 0,6 ml, plus préférentiellement entre 0,4 et 0,5 ml.

15. Utilisation selon la revendication 12, **caractérisée en ce que** la séquence d'ADN code pour un immunogène d'un agent pathogène choisi parmi le groupe consistant en :
virus BRSV, IBR, BVD, PI 3.

16. Utilisation selon l'une des revendications 13 à 15, **caractérisée en ce que** la formule de vaccin est destinée à être administrée par voie intradermique simultanément en de 1 à 10 points, notamment de 4 à 6, de préférence 5 ou 6.

## Patentansprüche

1. Polynucleotidimpfstoff-Formulierung für Rinder, die eine intradermal wirksame Menge eines Plasmids umfasst, das eine für ein Immunogen eines für Rinder pathogenen Organismus kodierende DNA-Sequenz und einen die *in-vivo*-Expression dieses Immunogens in den Hautzellen ermöglichenden Promotor verknüpft, wobei diese Impfstoffformulierung für die intradermale Verabreichung durch ein Gerät zur Abgabe mittels Flüssigkeitsstrahlen geeignet ist.

2. Impfstoffformulierung nach Anspruch 1, **dadurch gekennzeichnet, dass** das Plasmid in einem für den intradermalen Weg geeigneten Träger, der in der Lage ist, intradermal durch einen Flüssigkeitsstrahl verabreicht zu werden, mit einer Volumendosis von 0,1 bis 0,9 ml, vorzugsweise zwischen 0,2 und 0,6 ml, und besonders bevorzugt zwischen 0,4 und 0,5 ml vorliegt.

3. Impfstoffformulierung nach Anspruch 1 oder 2, **dadurch gekennzeichnet, dass** das Plasmid in der Impfstoffformulierung in einer intradermal wirksamen Menge von 10 ng bis 1 mg, vorzugsweise von 100 ng bis 500 µg, und besonders bevorzugt von 0,5 bis 50 µg vorliegt.

4. Impfstoffformulierung nach Anspruch 3, **dadurch gekennzeichnet, dass** die DNA-Sequenz für ein Immunogen eines pathogenen Organismus kodiert, der aus der Gruppe ausgewählt ist, die aus dem BRSV-Virus, IBR-Virus, BVD-Virus und PI-3-Virus besteht.

5. Impfstoffformulierung nach Anspruch 4, **dadurch gekennzeichnet, dass** die DNA-Sequenz für das Gen gB und/oder das Gen gD des IBR-Virus kodiert.

6. Impfstoffformulierung nach Anspruch 4, **dadurch gekennzeichnet, dass** die DNA-Sequenz für das Gen G und/oder F des BRSV-Virus kodiert.

7. Impfstoffformulierung nach Anspruch 4, **dadurch gekennzeichnet, dass** die DMA-Sequenz für E2 und/oder E1 des BVD-Virus kodiert.

8. Impfstoffformulierung nach Anspruch 4, **dadurch gekennzeichnet, dass** die DNA-Sequenz für HN und/oder F des PI-3-Virus kodiert.

9. Impfstoffformulierung nach einem der Ansprüche 1 bis 8, **dadurch gekennzeichnet, dass** der Promotor ein starker eukaryontischer Promotor wie der Promotor hCMV IE ist.

10. Impfstoffformulierung nach einem der Ansprüche 1 bis 9, **dadurch gekennzeichnet, dass** sie in einer Mehr-Dosen-Flasche aufbewahrt wird, die für ein Gerät zur intradermalen Verabreichung mittels Flüssigkeitsstrahlen, vorzugsweise den Pigjet, geeignet ist.

11. Tragbare Vorrichtung für das Impfen von Rindern, die ein Flüssigkeitsstrahlen erzeugendes Abgabegerät und eine geeignete Flasche, die mehrere Dosen einer Impfstoffformulierung nach einem der Ansprüche 1 bis 10 enthält, umfasst, wobei das Abgabegerät konstruiert ist, eine Dosis der Impfstoffformulierung intradermal abzugeben.

12. Vorrichtung nach Anspruch 11, **dadurch gekennzeichnet, dass** das Gerät einen Injektionskopf enthält, der mit 1 bis 10, insbesondere 4 bis 6, und vorzugsweise 5 oder 6 Düsen versehen ist.

13. Verwendung eines Plasmids, das eine für ein Immunogen eines für Rinder pathogenen Organismus kodierende DNA-Sequenz und einen die Expression dieses Immunogens ermöglichenden Promotor verknüpft, zur Herstellung einer Polynucleotidimpfstoff-Formulierung, die für die intradermale Verabreichung durch ein Gerät zur Abgabe mittels Flüssigkeitsstrahlen geeignet ist.

14. Verwendung nach Anspruch 13, **dadurch gekennzeichnet, dass** sie zwischen 10 ng und 1 mg, vorzugsweise zwischen 100 ng und 500 µg, und besonders bevorzugt zwischen 0,5 und 50 µg DNA in einer Volumendosis von 0,1 bis 0,9 ml, vorzugsweise zwischen 0,2 und 0,6 ml, und besonders bevorzugt zwischen 0,4 und 0,5 ml enthält.

15. Verwendung nach Anspruch 12, **dadurch gekennzeichnet, dass** die DNA-Sequenz für ein Immunogen eines pathogenen Organismus kodiert, der aus der Gruppe ausgewählt ist, die aus dem BRSV-, IBR-, BVD- und PI-3-Virus besteht.

16. Verwendung nach einem der Ansprüche 13 bis 15, **dadurch gekennzeichnet, dass** die Impfstoffformulierung an 1 bis 10, insbesondere 4 bis 6, und vorzugsweise 5 oder 6 Stellen gleichzeitig intradermal verabreicht werden soll,

## Claims

1. Polynucleotide vaccine formula intended for cattle comprising an intradermally effective amount of a plasmid combining a DNA sequence coding for an immunogen of a bovine pathogenic agent and a promoter which allows *in vivo* expression of this immunogen in the skin cells, this vaccine formula being adapted for intradermal administration by a liquid jet administration device.

2. Vaccine formula according to claim 1, **characterised in that** the plasmid is presented in a carrier adapted for intradermal administration in a dosage volume of between 0.1 and 0.9 ml, preferably between 0.2 and 0.6 ml, more preferably between 0.4 and 0.5 ml, suitable for intradermal administration by liquid jet.

3. Vaccine formula according to claim 1 or 2, **characterised in that** the plasmid is present in the vaccine formula in an intradermally effective amount of from 10 ng to 1 mg, preferably from 100 ng to 500 µg, more preferably from 0.5 µg to 50 µg.

4. Vaccine formula according to claim 3, **characterised in that** the DNA sequence codes for an immunogen of a pathogenic agent selected from among BRSV virus, IBR virus, BVD virus and PI 3 virus.

5. Vaccine formula according to claim 4, **characterised in that** the DNA sequence codes for the gene gB and/or the gene gD of the IBR virus.

6. Vaccine formula according to claim 4, **characterised in that** the DNA sequence codes for the gene G and/or F of the BRSV virus.

7. Vaccine formula according to claim 4, **characterised in that** the DNA sequence codes for E2 and/or E1 of the BVD virus.

8. Vaccine formula according to claim 4, **characterised in that** the DNA sequence codes for HN and/or F of the virus PI 3.

9. Vaccine formula according to any one of claims 1 to 8, **characterised in that** the promoter is a strong eukaryotic promoter such as the promoter hCMV IE.

10. Vaccine formula according to any one of claims 1 to 9, **characterised in that** it is packaged in a multi-dose bottle adapted to be used with a liquid jet intradermal administration device, preferably the Pigjet.

11. Portable cattle vaccination apparatus comprising a liquid jet administration device and a suitable bottle containing several doses of a vaccine formula according to any one of claims 1 to 10, the administration device being designed to deliver a dose of vaccine formula by intradermal route.

12. Apparatus according to claim 11, **characterised in that** the device comprises an injection head fitted with 1 to 10, notably 4 to 6, preferably 5 or 6 nozzles.

13. Use of a plasmid combining a DNA sequence coding for an immunogen of a bovine pathogenic agent and a promoter allowing expression of this immunogen, in order to prepare a polynucleotide vaccine formula adapted for intradermal administration by a liquid jet administration device.

14. Use according to claim 13, **characterised in that** it comprises between 10 ng and 1 mg of DNA, preferably between 100 ng and 500 µg, preferably between 0.5 µg and 50 µg, in a dosage volume of between 0.1 and 0.9 ml, preferably between 0.2 and 0.6 ml, more preferably between 0.4 and 0.5 ml.

15. Use according to claim 12, **characterised in that** the DNA sequence codes for an immunogen of a pathogenic agent selected from among BRSV, IBR, BVD and PI 3 virus.

16. Use according to one of claims 13 to 15, **characterised in that** the vaccine formula is intended to be administered by intradermal route at 1 to 10, notably 4 to 6, preferably 5 or 6 points simultaneously.
